(19) Europäisches Patentamt
European Patent Office
Office européen des brevets

(11) Veröffentlichungsnummer: **0 384 246 A2**

(12) # EUROPÄISCHE PATENTANMELDUNG

(21) Anmeldenummer: **90102638.5**

(22) Anmeldetag: **10.02.90**

(51) Int. Cl.[5]: **C07D 249/08, C07D 303/18, C07D 303/40, A01N 43/653**

(30) Priorität: **21.02.89 DE 3905316**

(43) Veröffentlichungstag der Anmeldung: **29.08.90 Patentblatt 90/35**

(84) Benannte Vertragsstaaten: **BE CH DE DK FR GB IT LI NL**

(71) Anmelder: **BAYER AG**

**D-5090 Leverkusen 1 Bayerwerk(DE)**

(72) Erfinder: **Jäger, Gerhard, Dr.**
**Paul-Klee-Srasse 68j**
**D-5090 Leverkusen(DE)**
Erfinder: **Kraatz, Udo, Dr.**
**Andreasstrasse 22a**
**D-5090 Leverkusen(DE)**
Erfinder: **Dutzmann, Stefan, Dr.**
**Leinenweberweg 33**
**D-4000 Duesseldorf(DE)**
Erfinder: **Brandes, Wilhelm, Dr.**
**Eichendorffstrasse 3**
**D-5653 Leichlingen 1(DE)**
Erfinder: **Hänssler, Gerd, Dr.**
**Am Arenzberg 58a**
**D-5090 Leverkusen 3(DE)**

(54) **Cyclopropyl-hydroxyethyl-azolyl-Derivate.**

(57) Neue Cyclopropyl-hydroxyethyl-azolyl-Derivate der Formel

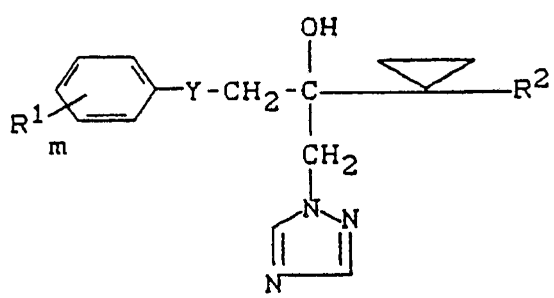

(I)

in welcher
$R^1$, $R^2$, Y und m die in der Beschreibung angegebene Bedeutung haben,
sowie deren Säureadditions-Salze und Metallsalz-Komplexe, ein Verfahren zur Herstellung der neuen Stoffe und deren Verwendung als Fungizide und Pflanzenwuchsregulatoren.
Neue Oxirane der Formel

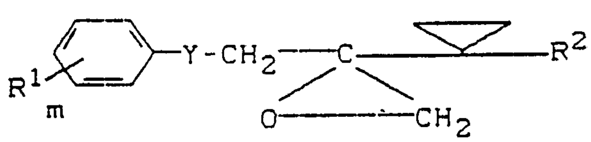

(II)

und ein Verfahren zu deren Herstellung.

EP 0 384 246 A2

## Cyclopropyl-hydroxyethyl-azolyl-Derivate

Die vorliegende Erfindung betrifft neue Cyclopropyl-hydroxyethyl-azolyl-Derivate, ein Verfahren zu deren Herstellung und deren Verwendung als Fungizide und Pflanzenwuchsregulatoren.

Es ist bereits bekannt, daß zahlreiche Hydroxyalkyl-azolyl-Derivate fungizide und pflanzenwuchsregulierende Eigenschaften besitzen (vgl. EP-OS 0 040 345 und EP-OS 0 297 383). Die Wirksamkeit dieser Stoffe ist sehr gut; bei niedrigen Aufwandmengen läßt die Wirksamkeit allerdings in manchen Fällen zu wünschen übrig.

Es wurden nun neue Cyclopropyl-hydroxyethyl-azolyl-Derivate der Formel

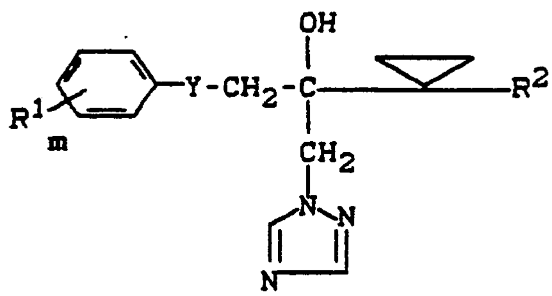

(I)

in welcher

$R^1$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Alkoximinomethyl mit 1 bis 4 Kohlenstoffatomen in der Alkoxygruppe, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,

m für die Zahlen 0, 1, 2 oder 3 steht,

$R^2$ für gegebenenfalls substituiertes Benzyl oder für den Rest $-COOR^3$ steht, worin

$R^3$ für Alkyl, gegebenenfalls substituiertes Phenyl oder gegebenenfalls substituiertes Benzyl steht,

und

Y für Sauerstoff oder Schwefel steht,

sowie deren Säureadditions-Salze und Metallsalz-Komplexe gefunden.

Die erfindungsgemäßen Stoffe enthalten ein asymmetrisch substituiertes Kohlenstoffatom. Sie können daher in optischen Isomerenformen anfallen. Die Erfindung betrifft sowohl die einzelnen Isomeren als auch deren Gemische.

Weiterhin wurde gefunden, daß man Cyclopropyl-hydroxyethyl-azolyl-Derivate der Formel (I) sowie deren Säureadditions-Salze und Metallsalz-Komplexe erhält, wenn man Oxirane der Formel

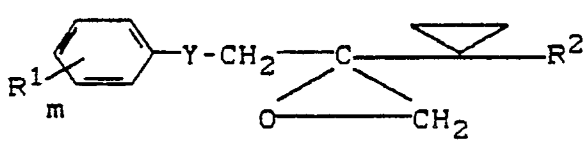

(II)

in welcher

$R^1$, $R^2$,Y und m die oben angegebene Bedeutung haben,

mit 1,2,4-Triazol der Formel

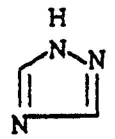

(III)

in Gegenwart eines Säurebindemittels und in Gegenwart eines Verdünnungsmittels umsetzt,
und gegebenenfalls anschließend an die so erhaltenen Verbindungen der Formel (I) eine Säure oder ein Metallsalz addiert.

Schließlich wurde gefunden, daß die neuen Cyclopropyl-hydroxyethyl-azolyl-Derivate der Formel (I) sowie deren Säureadditions-Salze und Metallsalz-Komplexe starke fungizide und pflanzenwuchsregulierende Eigenschaften besitzen.

Überraschenderweise besitzen die erfindungsgemäßen Stoffe eine bessere fungizide und pflanzenwuchsregulierende Wirksamkeit als die konstitutionell ähnlichsten vorbekannten Verbindungen gleicher Wirkungsrichtung.

Die erfindungsgemäßen Hydroxyethyl-azolyl-Derivate sind durch die Formel (I) allgemein definierte. In dieser Formel stehen vorzugsweise
$R^1$ für Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl gegebenenfalls durch Fluor, Chlor und/oder Methyl substituiertes Phenyl oder für gegebenenfalls durch Fluor, Chlor und/oder Methyl substituiertes Phenoxy,
m für die Zahlen 0, 1, 2 oder 3,
$R^2$ für Benzyl, das im Phenylteil gegebenenfalls einfach bis dreifach, gleichartig oder verschieden durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist, oder
$R^2$ für den Rest der Formel $-COOR^3$, worin
$R^3$ für Alkyl mit 1 bis 4 Kohlenstoffatomen, gegebenenfalls durch Halogen und/oder Alkyl mit 1 bis 4 Kohlenstoffatomen substituiertes Phenyl oder für gegebenenfalls durch Halogen und/oder Alkyl mit 1 bis 4 Kohlenstoffatomen substituiertes Benzyl steht,
und
Y für Sauerstoff oder Schwefel.

Besonders bevorzugt sind diejenigen Verbindungen der Formel (I), in denen
$R^1$ für Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, gegebenenfalls durch Fluor, Chlor und/oder Methyl substituiertes Phenyl oder für gegebenenfalls durch Fluor, Chlor und/oder Methyl substituiertes Phenoxy,
m für die Zahlen 0, 1, 2 oder 3,
$R^2$ für Benzyl steht, das im Phenylteil gegebenenfalls einfach oder zweifach, gleichartig oder verschieden durch Fluor, Chlor, Brom, Trifluormethyl, Methyl, Ethyl, Methoxy und/oder Ethoxy substituiert ist, oder
$R^2$ für den Rest der Formel $-COOR^3$ steht, worin
$R^3$ für Methyl, Ethyl, n-Propyl, Isopropyl, n-Butyl, tert.-Butyl, gegebenenfalls durch Fluor, Chlor und/oder Methyl substituiertes Phenyl oder für gegebenenfalls durch Fluor, Chlor und/oder Methyl substituiertes Benzyl steht,
und
Y für Sauerstoff oder Schwefel steht.

Bevorzugte erfindungsgemäße Verbindungen sind auch Additionsprodukte aus Säuren und denjenigen Cyclopropyl-hydroxyethyl-azolyl-Derivaten der Formel (I), in denen $R^1$, $R^2$, Y und m die Bedeutungen haben, die bereits vorzugsweise für diese Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I und II. sowie IV. bis VIII. Nebengruppe des Periodensystems der Elemente und denjenigen Cyclopropyl-hydroxyethyl-azolyl-Derivaten der Formel (I), in denen $R^1$, $R^2$, Y und m die Bedeutungen haben, die bereits vorzugsweise für diese Reste und diesen Index genannt wurden.

Hierbei sind die Salze des Kupfers, Zinks, Mangans, Magnesiums, Zinns, Eisens und des Nickels besonders bevorzugt. Als Anionen dieser Salze kommen solche in Betracht, die sich von solchen Säuren

ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man 2-[1-(4-Chlor-benzyl)-cyclopropyl]-2-(4-chlorphenoxy-methyl)-oxiran und 1,2,4-Triazol als Ausgangsstoffe, so kann der Verlauf des erfindungsgemäßen Verfahrens durch das folgende Formelschema veranschaulicht werden:

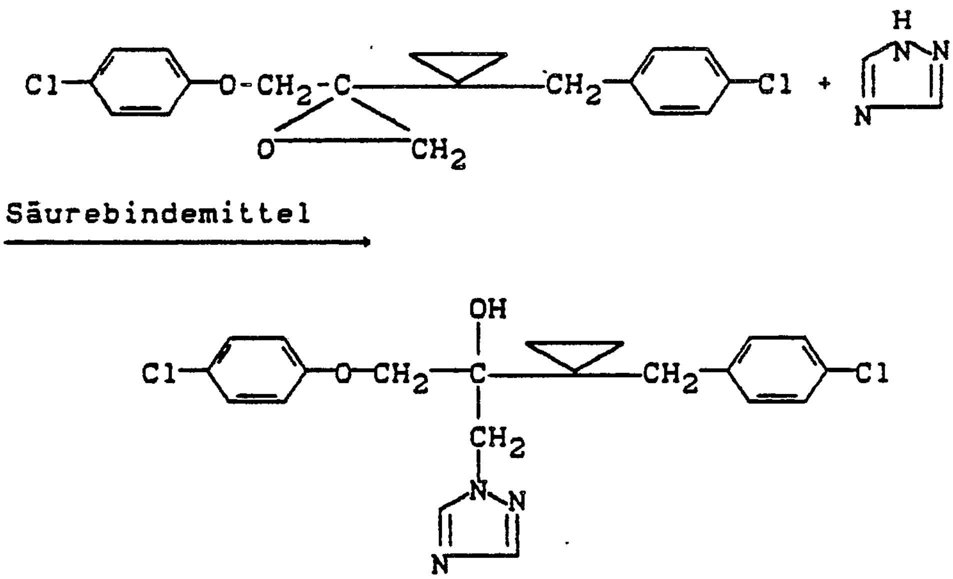


Die bei dem erfindungsgemäßen Verfahren als Ausgangsstoffe benötigten Oxirane sind durch die Formel (II) allgemein definiert. In dieser Formel haben $R^1$, $R^2$, Y und m vorzugsweise diejenigen Bedeutungen, die bereits im Zusammenhang mit der Beschreibung der erfindungsgemäßen Stoffe der Formel (I) vorzugsweise für diese Reste und für diesen Index genannt wurden.

Die Oxirane der Formel (II) sind bisher noch nicht bekannt. Sie lassen sich herstellen, indem man

a) Cyclopropylketone der Formel

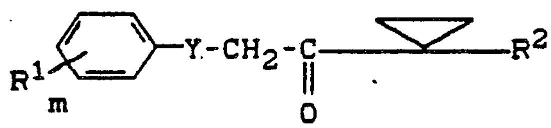
(IV)

in welcher

$R^1$, $R^2$, Y und m die oben angegebene Bedeutung haben,

entweder

α) mit Dimethyloxosulfonium-methylid der Formel

$(CH_3)_2 \overset{\delta\oplus}{S}O\overset{\delta\ominus}{C}H_2$ (V)

oder

β) mit Dimethylsulfonium-methylid der Formel

$(CH_3)_2 \overset{\delta\oplus}{S}\ \overset{\delta\ominus}{C}H_2$ (VI) in Gegenwart eines Verdünnungsmittels umsetzt.

Die bei dem Verfahren (a) als Ausgangsstoffe benötigten Cyclopropylketone der Formel (IV) lassen sich herstellen, indem man

b) Halogenketone der Formel

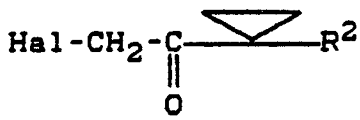

(VII)

in welcher
$R^2$ die oben angegebene Bedeutung hat und
Hal für Chlor oder Brom steht,
mit Phenyl-Derivaten der Formel

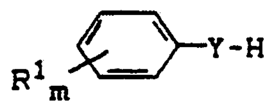

(VIII)

in welcher
$R^1$, Y und m die oben angegebene Bedeutung haben,
in Gegenwart eines Säurebindemittels und gegebenenfalls in Gegenwart eines Verdünnungsmittels umsetzt.

Die bei dem Verfahren (b) als Ausgangsstoffe benötigten Halogenketone der Formel (VII) lassen sich herstellen, indem man

c) Ketone der Formel

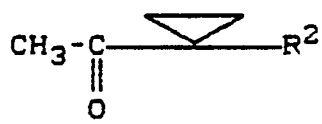

(IX)

in welcher
$R^2$ die oben angegebene Bedeutung hat,
mit Chlorierungsmitteln oder Bromierungsmitteln in Gegenwart eines Verdünnungsmittels umsetzt.

Die bei dem Verfahren (c) als Ausgangsstoffe benötigten Ketone der Formel (IX) sind bekannt oder lassen sich nach prinzipiell bekannten Verfahren synthetisieren (vgl. Synthesis 1977, 189).

Als Chlorierungsmittel und Bromierungsmittel kommen bei dem Verfahren (c) alle für derartige Umsetzungen üblichen Chlorierungs- und Bromierungsreagenzien in Betracht. Vorzugsweise verwendbar sind Sulfurylchlorid, Sulfurylbromid, Chlor und Brom.

Als Verdünnungsmittel kommen bei dem Verfahren (c) alle für derartige Umsetzungen üblichen inerten organischen Solventien in Frage. Vorzugsweise verwendbar sind halogenierte aliphatische Kohlenwasserstoffe, wie Methylenchlorid, Chloroform und Tetrachlorkohlenstoff.

Die Reaktionstemperaturen können bei dem Verfahren (c) innerhalb eines bestimmten Bereiches variiert werden. Im allgemeinen arbeitet man bei Temperaturen zwischen -10 °C und +60 °C, vorzugsweise zwischen 0 °C und +40 °C.

Bei der Durchführung des Verfahrens (c) arbeitet man ebenso wie bei den übrigen in dieser Anmeldung beschrieben Verfahren im allgemeinen unter Normaldruck. Es ist jedoch jeweils auch möglich, unter erhöhtem oder vermindertem Druck zu arbeiten.

Bei der Durchführung des Verfahrens (c) setzt man auf 1 Mol an Keton der Formel (IX) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß man das Reaktionsgemisch nacheinander mit verdünnter, wäßriger Natriumhydrogencarbonat-Lösung und Wasser wäscht, dann trocknet und einengt.

Die bei dem Verfahren (b) als Reaktionskomponenten benötigten Phenyl-Derivate sind durch die Formel (VIII) allgemein definiert. In dieser Formel haben $R^1$, Y und m vorzugsweise diejenigen Bedeutungen, die bereits im Zusammenhang mit der Beschreibung der erfindungsgemäßen Stoffe der Formel (I) vorzugsweise für diese Reste und für diesen Index genannt wurden. - Die Phenyl-Derivate der Formel (VIII) sind allgemein bekannte Verbindungen der organischen Chemie.

Als Säurebindemittel kommen bei der Durchführung des Verfahrens (b) alle üblichen Säureakzeptoren in Betracht. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie

Natriumhydroxid, Kaliumhydroxid, Natriummethylat oder Kaliumtert.-butylat, außerdem tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo [4.3.0]non-5-en (DBN), 1,8-Diaza-bicyclo-[5.4.0]undec-7-en (DBU) und 1,4-Diaza-bicyclo[2.2.2]octan (DABCO).

Als Verdünnungsmittel kommen bei der Durchführung des Verfahrens (b) alle inerten organischen Solventien in Betracht. Vorzugsweise verwendbar sind aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril und Pyridin, sowie auch stark polare Lösungsmittel, wie Dimethylformamid und Dimethylsulfoxid.

Die Reaktionstemperaturen können bei dem Verfahren (b) innerhalb eines größeren Bereiches variiert werden. Im allgemeinen arbeitet man bei Temperaturen zwischen 0 °C und 150 °C, vorzugsweise zwischen 20 °C und 130 °C.

Bei der Durchführung des Verfahrens (b) setzt man auf 1 Mol an Halogenketon der Formel (VII) im allgemeinen 1 bis 1,5 Mol an Phenyl-Derivat der Formel (VIII) sowie 1 bis 2 Mol an Säurebindemittel ein. Die Aufarbeitung erfolgt nach üblichen Methoden. Im allgemeinen geht man so vor, daß man gegebenenfalls nach vorherigem Abfiltrieren von abgeschiedenen Salzen das Reaktionsgemisch einengt, den Rückstand in einem mit Wasser wenig mischbaren organischen Lösungsmittel aufnimmt, die entstehende Lösung wäscht, trocknet und dann einengt.

Das bei dem Verfahren (a) als Reaktionskomponente benötigte Dimethyl-oxo-sulfonium-methylid der Formel (V) ist bekannt (vgl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, Kalium-tert.-butylat oder Natriummethylat, in Gegenwart eines Verdünnungsmittels erzeugt.

Das bei dem Verfahren (a) außerdem als Reaktionskomponente in Betracht kommende Dimethylsulfonium-methylid der Formel (VI) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfoniumhalogenid oder Trimethylsulfoniummethylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (a) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid oder Acetonitril.

Die Reaktionstemperaturen können bei der Durchführung des Verfahrens (a) in einem größeren Bereich variiert werden. Im allgemeinen arbeitet man zwischen 0 °C und 100 °C, vorzugsweise zwischen 10 °C und 60 °C.

Bei der Durchführung des Verfahrens (a) setzt man auf 1 Mol an Cyclopropyl-keton der Formel (IV) im allgemeinen 1 bis 3 Mol an Dimethyloxosulfonium-methylid der Formel (V) bzw. an Dimethylsulfonium-methylid der Formel (VI) ein. Die Isolierung der Oxirane der Formel (II) erfolgt nach üblichen Methoden.

Als Säurebindemittel kommen bei der Durchführung des erfindungsgemäßen Verfahrens alle üblichen Säureakzeptoren in Frage. Vorzugsweise verwendbar sind alle diejenigen Säurebindemittel, die bereits im Zusammenhang mit der Beschreibung des Verfahrens (b) vorzugsweise genannt wurden.

Als Verdünnungsmittel kommen bei der Durchführung des erfindungsgemäßen Verfahrens alle üblichen inerten organischen Solventien in Betracht. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsäuretriamid.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 200 °C, vorzugsweise zwischen 50 °C und 150 °C.

Bei der Durchführung des erfindungsgemäßen Verfahrens setzt man vorzugsweise auf 1 Mol an Oxiran der Formel (II), 1 bis 4 Mol an 1,2,4-Triazol der Formel (III) und 1 bis 2 Mol an Base ein. Die Isolierung der Endprodukte erfolgt in üblicher Weise.

Die nach dem erfindungsgemäßen Verfahren erhältlichen Cyclopropyl-hydroxyethyl-azolyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise

diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Rost, Mehltau Cochliobolus sativus, Pyrenophora teres und Leptosphaeria nodorum an Getreide, oder Pyricularia oryzae an Reis. Ferner lassen sie sich gegen Sphaerotheca an Gurken verwenden.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb

als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu

ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin- Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwi schen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

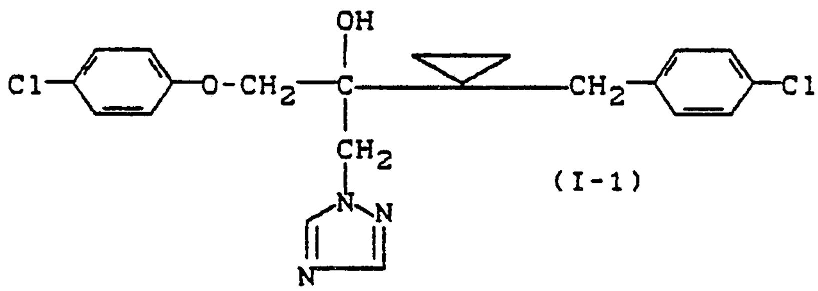

(I-1)

Zur Suspension von 13,4 g (0,2 Mol) 1,2,4-Triazol und 26,7 g (0,2 Mol) Kaliumcarbonat in 150 ml Dimethylformamid fügt man 28 g (0,08 Mol) 2-(4-Chlorphenoxymethyl)-2-(4-chlorbenzyl-cycloprop-1-yl)-oxiran und heizt unter Rühren auf 90° C. Nach 16-stündigem Rühren entfernt man das Dimethylformamid weitgehend unter vermindertem Druck bei 45° C und behandelt den Rückstand mit Methylenchlorid/Wasser. Die organische Phase wird abgetrennt, zweimal mit Wasser gewaschen, über Magnesiumsulfat getrocknet und unter vermindertem Druck eingeengt. Der Rückstand wird aus Isopropanol umkristallisiert.
Ausbeute: 23 g (70 % der Theorie)
Fp. 155° C.

Herstellung von Ausgangssubstanzen:

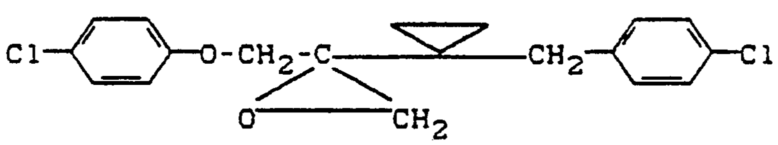

(II-2)

21 g (0,17 Mol) Dimethylsulfat werden unter Rühren bei 20° C zu einer Lösung von 10,5 g (0,17 Mol)

Dimethylsulfid in 100 ml Acetonitril getropft. Nach 2 Tagen Stehen bei 20°C fügt man dieser Lösung 9,2 g (0,17 Mol) Natriummethylat zu, rührt 30 Minuten bei 20°C und versetzt dann mit 29 g (0,09 Mol) (4-Chlorphenoxymethyl)-[1-(4-chlorbenzyl)-cycloprop-1-yl]-keton. Nach 16-stündigem Rühren bei 20°C gießt man das Reaktionsgemisch in Wasser und extrahiert mit Methylenchlorid. Die organische Phase wird mit Wasser gewaschen, über Magnesiumsulfat getrocknet und unter vermindertem Druck eingeengt. Man erhält 25,2 g (80 % der Theorie) an 2-(4-Chlorphenoxymethyl)-2-[(4-chlorbenzyl )cycloprop-1-yl]-oxiran in Form eines farblosen Öles, das ohne zusätzliche Reinigung für die weitere Synthese eingesetzt wird.

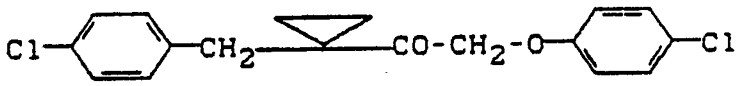

(IV-1)

26,1 g (0,09 Mol) 1-(4-Chlorbenzyl)-1-bromacetyl-cyclopropan werden unter Rühren bei 20°C in eine Lösung von 11,6 g (0,09 Mol) 4-Chlorphenol und 12,4 g (0,09 Mol) Kaliumcarbonat in 150 ml Aceton getropft. Nach 16-stündigem Erhitzen unter Rückfluß werden die anorganischen Bestandteile abfiltriert. Das Filtrat wird unter vermindertem Druck eingeengt, und der Rückstand wird mit Wasser/Methylenchlorid aufgenommen. Die organische Phase wird zweimal mit 1N-Natronlauge gewaschen und unter vermindertem Druck eingeengt. Man erhält (4-Chlorphenoxymethyl)-[1-(4-chlorbenzyl)-cycloprop-1-yl]-keton als zähes Öl, das ohne weitere Reinigung umgesetzt wird. Ausbeute 27,4 g (91 % der Theorie).

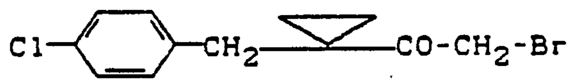

(VII-1)

Zu einer Lösung von 62,6 g (0,3 Mol) 1-(4-Chlorbenzyl)-1-acetyl-cyclopropan in 200 ml Methanol werden bei -10°C 48 g (0,3 Mol) Brom so zugetropft, daß immer eine Entfärbung auftritt. Man rührt dann noch 1 Stunde bei 20°C nach, gießt das Gemisch in 600 ml Wasser und extrahiert mit Methylenchlorid. Die organische Phase wird mit Wasser gewaschen, über Magnesiumsulfat getrocknet und unter vermindertem Druck eingeengt. Man erhält so 81,8 g an 1-(4-Chlorbenzyl)-1-bromacetylcyclopropan in Form einer öligen Flüssigkeit. Die Ausbeute errechnet sich danach zu 95 % der Theorie. Das Produkt wird ohne zusätzliche Reinigung für die weitere Synthese verwendet.

Beispiel 2

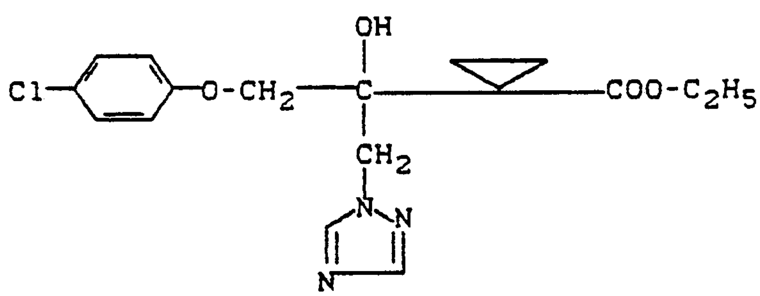

(I-2)

Zu einer Lösung von 1,6 g (0,07 Mol) Natrium in 10 ml Butanol fügt man 5,4 g (0,077 Mol) 1,2,4-Triazol und 20,8 g (0,07 Mol) 2-[(4-Chlorphenoxy)-methyl]-2-(1-ethoxycarbonyl-cycloprop-1-yl)-oxiran hinzu. Nach 14-stündigem Kochen unter Rückfluß wird das Lösungsmittel unter vermindertem Druck entfernt und der Rückstand mit Methylenchlorid/Wasser aufgenommen. Die organische Phase wird abgetrennt, über Magnesiumsulfat getrocknet und unter vermindertem Druck eingeengt. Der Rückstand wird an Kieselgel mit Chloroform chromatographiert.
Ausbeute 8,6 g (33,6 % der Theorie)
Fp. 56-58°C.

Herstellung von Ausgangssubstanzen:

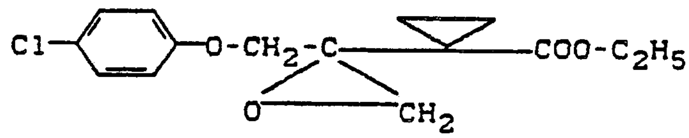

(II-2)

Zu einer Suspension von 24 g (0,11 Mol) Trimethylsulfoxoniumiodid in 100 ml Dimethylsulfoxid fügt man unter Argonatmosphäre und unter Rühren bei 10 bis 20 °C 3,6 g (0,13 Mol) 80 %iges Natriumhydrid hinzu. Nach 30-minütigem Rühren bei 20 °C werden 17,1 g (0,061 Mol) 4-Chlorphenoxymethyl-(1-ethoxycarbonylcycloprop-1-yl)-keton zugegeben. Das Gemisch wird 1 Stunde auf 60 °C erwärmt. Danach gießt man das Gemisch in 300 ml Wasser und extrahiert mit Methylenchlorid. Die vereinigten organischen Extrakte werden mit Wasser gewaschen, über Magnesiumsulfat getrocknet und eingeengt. Man erhält 14 g (77,8 % der Theorie) an 2-[(4-Chlorphenoxy)-methyl]-2-(1-ethoxycarbonyl-cycloprop-1-yl)-oxiran als Öl, das ohne zusätzliche Reinigung weiter umgesetzt wird.

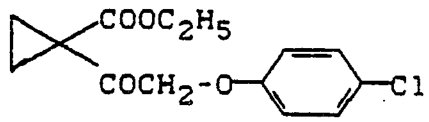

(IV-2)

Zur Suspension von 28 g (0,2 Mol) Kaliumcarbonat und 26 g (0,2 Mol) 4-Chlorphenol in 300 ml Aceton fügt man 47,2 g (0,2 Mol) 1-(Bromacetyl)-cyclopropancarbonsäureethylester und erhitzt 6 Stunden unter Rückfluß. Anschließend saugt man von anorganischen Salzen ab, engt das Filtrat unter vermindertem Druck ein und destilliert den Rückstand in Vakuum. Man erhält 39 g (69 % der Theorie) an 1-(4-Chlorphenoxyacetyl)-cyclopropancarbonsäureethylester.

Kp. 148-150 °C/0,45 mbar.

Nach den zuvor beschriebenen Methoden werden auch die in der folgenden Tabelle 1 aufgeführten Stoffe der Formel

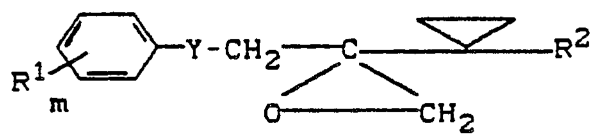

(II)

hergestellt.

<u>Tabelle 1</u>

| Bsp. Nr. | Verb. Nr. | $R^1_m$ | $R^2$ | Y | Schmelzpunkt (°C) |
|---|---|---|---|---|---|
| 3 | I-3 | 4-$CH_3$ | -$CH_2$ 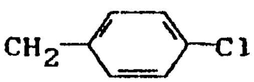 | O | 116 |
| 4 | I-4 | 4-Cl | -$CH_2$ 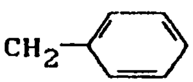 | O | 87 |
| 5 | I-5 | 4-Cl | -$CH_2$ 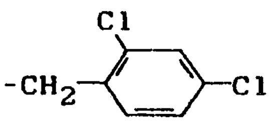 | O | 118-123 |
| 6 | I-6 | 2-$CH_3$, 4-Cl | -$CH_2$ 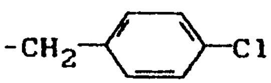 | O | 92 |
| 7 | I-7 | 4-F | -$CH_2$ 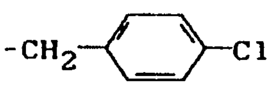 | O | 125 |
| 8 | I-8 | 4-$OCF_3$ | -$CH_2$ 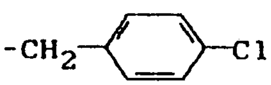 | O | 123 |
| 9 | I-9 | 4-Br | -COO-$C_2H_5$ | O | 58-59 |

Tabelle 1

| Bsp. Nr. | Verb. Nr. | $R^1_m$ | $R^2$ | Y | Schmelzpunkt (°C) |
|---|---|---|---|---|---|
| 10 | I-10 | 4-F | $-COO-C_2H_5$ | O | 57-58 |
| 11 | I-11 | 2,4-$Cl_2$ | $-CH_2-$ 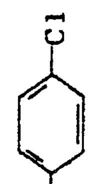 | O | 104 |

Verwendungsbeispiele

In den folgenden Beispielen wurden die nachstehend angegebenen Verbindungen als Vergleichssubstanzen eingesetzt:

(A) = 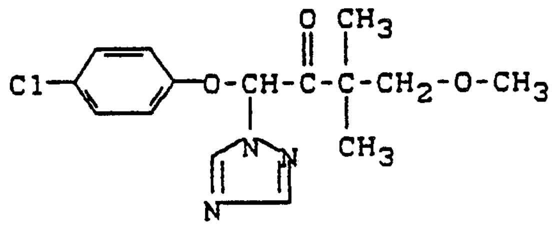

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-9) und (I-10) eine sehr gute Wirksamkeit.

Beispiel B

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigt die erfindungsgemäße Verbindung (I-2) eine sehr gute Wirksamkeit.

Beispiel C

Venturia-Test (Apfel) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

In diesem Test zeigen die erfindungsgemäßen Verbindungen (I-2), (I-9) und (I-10) eine wesentlich bessere Wirksamkeit als die Vergleichssubstanz (A).

Beispiel D

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

In diesem Test zeigen die erfindungsgemäßen Verbindungen (I-6), (I-8) und (I-9) eine sehr gute Wirksamkeit.

Beispiel E

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25° C und einer rel. Luftfeuchtigkeit von 100 % bis zur Auswertung.

4 Tage nach der Inokulation erfolgt die Auswertung des Krankheitsbefalls.

In diesem Test zeigen die erfindungsgemäßen Verbindungen (I-2) und (I-10) eine sehr gute Wirksamkeit.

**Ansprüche**

1. Cyclopropyl-hydroxyethyl-azolyl-Derivate der Formel

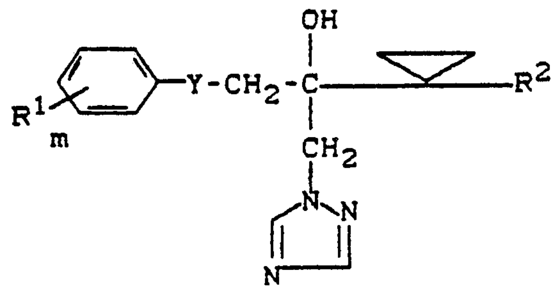

(I)

in welcher
$R^1$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Alkoximinomethyl mit 1 bis 4 Kohlenstoffatomen in der Alkoxygruppe, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,
m für die Zahlen 0, 1, 2 oder 3 steht,
$R^2$ für gegebenenfalls substituiertes Benzyl oder für den Rest $-COOR^3$ steht, worin
$R^3$ für Alkyl, gegebenenfalls substituiertes Phenyl oder gegebenenfalls substituiertes Benzyl steht,
und
Y für Sauerstoff oder Schwefel steht,
sowie deren Säureadditions-Salze und Metallsalz-Komplexe.

2. Verfahren zur Herstellung von Cyclopropyl-hydroxyethyl-azolyl-Derivaten der Formel

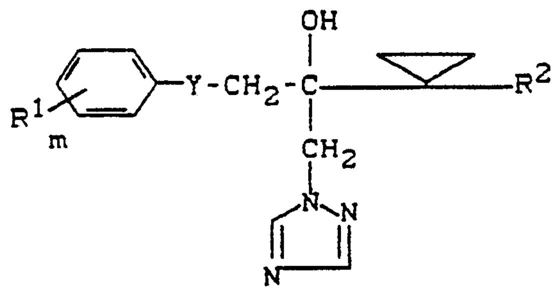

(I)

in welcher
$R^1$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Alkoximinomethyl mit 1 bis 4 Kohlenstoffatomen in der Alkoxygruppe, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,
m für die Zahlen 0, 1, 2 oder 3 steht,
$R^2$ für gegebenenfalls substituiertes Benzyl oder für den Rest $-COOR^3$ steht, worin
$R^3$ für Alkyl, gegebenenfalls substituiertes Phenyl oder gegebenenfalls substituiertes Benzyl steht,
und
Y für Sauerstoff oder Schwefel steht,
sowie von deren Säureadditions-Salzen und Metallsalz-Komplexen, dadurch gekennzeichnet, daß man Oxirane der Formel

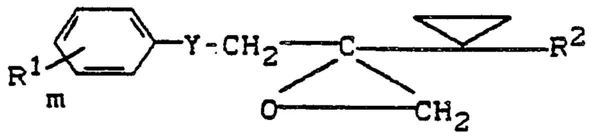

(II)

in welcher
$R^1$, $R^2$,Y und m die oben angegebene Bedeutung haben,
mit 1,2,4-Triazol der Formel

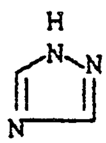

(III)

in Gegenwart eines Säurebindemittels und in Gegenwart eines Verdünnungsmittels umsetzt, und gegebenenfalls anschließend an die so erhaltenen Verbindungen der Formel (I) eine Säure oder ein Metallsalz addiert.

3. Fungizide und pflanzenwuchsregulierende Mittel, gekennzeichnet durch einen Gehalt an mindestens einem Cyclopropyl-hydroxyethyl-azolyl-Derivat der Formel (I) gemäß Anspruch 1 bzw. an einem Säureadditions-Salz oder Metallsalz-Komplex eines Cyclopropyl-hydroxyethyl-azolyl-Derivates der Formel (I).

4. Verwendung von Cyclopropyl-hydroxyethyl-azolyl-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

5. Verfahren zur Bekämpfung von Pilzen und zur Regulierung des Pflanzenwachstums, dadurch gekennzeichnet, daß man Cyclopropyl-hydroxyethyl-azolyl-Derivate der Formel (I) gemäß Anspruch 1 bzw. deren Säureadditions-Salze oder Metallsalz-Komplexe auf die Pflanzen und/oder deren Lebensraum ausbringt.

6. Verfahren zur Herstellung von fungiziden und pflanzenwuchsregulierenden Mitteln, dadurch gekennzeichnet, daß man Cyclopropyl-hydroxyethyl-azolyl-Derivate der Formel (I) gemäß Anspruch 1 bzw. deren Säureadditions-Salze oder Metallsalz-Komplexe mit Streckmitteln und/oder oberflächenaktiven Stoffen vermischt.

7. Oxirane der Formel

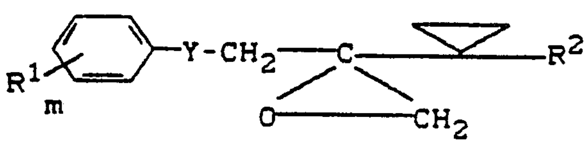

(II)

in welcher
$R^1$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Alkoximinomethyl mit 1 bis 4 Kohlenstoffatomen in der Alkoxygruppe, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,
m für die Zahlen 0, 1, 2 oder 3 steht,
$R^2$ für gegebenenfalls substituiertes Benzyl oder für den Rest $-COOR^3$ steht, worin
$R^3$ für Alkyl, gegebenenfalls substituiertes Phenyl oder gegebenenfalls substituiertes Benzyl steht,
und
Y für Sauerstoff oder Schwefel steht.

8. Verfahren zur Herstellung von Oxiranen der Formel

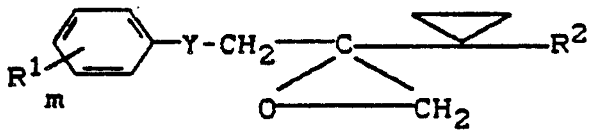

(II)

in welcher
$R^1$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Alkoximinomethyl mit 1 bis 4 Kohlenstoffatomen in der Alkoxygruppe, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht,
m für die Zahlen 0, 1, 2 oder 3 steht,
$R^2$ für gegebenenfalls substituiertes Benzyl oder für den Rest -$COOR^3$ steht, worin
$R^3$ für Alkyl, gegebenenfalls substituiertes Phenyl oder gegebenenfalls substituiertes Benzyl steht,
und
Y für Sauerstoff oder Schwefel steht,
dadurch gekennzeichnet, daß man
a) Cyclopropylketone der Formel

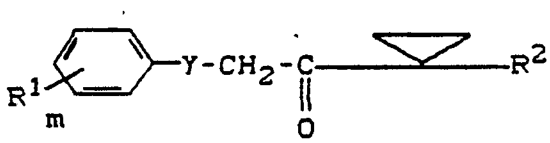

(IV)

in welcher
$R^1$, $R^2$, Y und m die oben angegebene Bedeutung haben,
entweder
α) mit Dimethyloxosulfonium-methylid der Formel

$$(CH_3)_2 \overset{\delta\oplus}{S}O\overset{\delta\ominus}{C}H_2 \quad (V)$$

oder
ß) mit Dimethylsulfonium-methylid der Formel

$$(CH_3)_2 \overset{\delta\oplus}{S} \quad \overset{\delta\ominus}{C}H_2 \quad (VI)$$

in Gegenwart eines Verdünnungsmittels umsetzt.

9. Cyclopropyl-hydroxyethyl-azolyl-Derivat gemäß Anspruch 1, gekennzeichnet durch die Formel

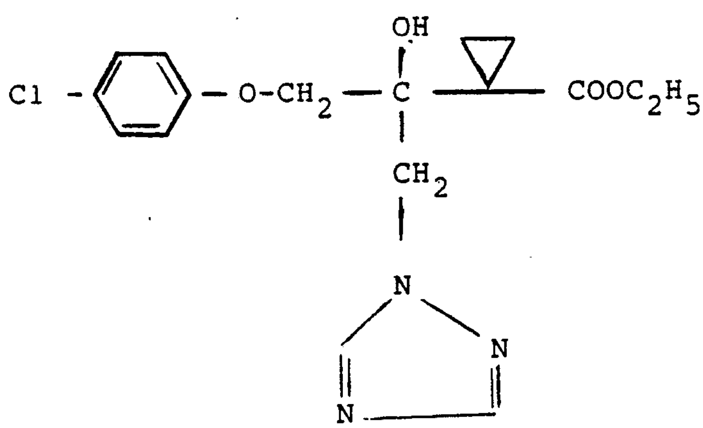

10. Cyclopropyl-hydroxyethyl-azolyl-Derivat gemäß Anspruch 1, gekennzeichnet durch die Formel